# EUROPEAN PATENT SPECIFICATION

(11) **EP 3 011 060 B1**
(45) Date of publication and mention of the grant of the patent: **04.04.2018**
(21) Application number: 14733587.1
(22) Date of filing: 19.06.2014
(51) Int. Cl.: C12Q 1/70

(54) **A METHOD OF CERVICAL SCREENING**
VERFAHREN ZUR GEBÄRMUTTERHALSABTASTUNG
PROCÉDÉ D'ANALYSE DE L'ÉTAT DU COL DE L'UTÉRUS

(30) Priority: 19.06.2013 GB 201310933
(43) Date of publication of application: 27.04.2016
(73) Proprietor: Norchip AS, 3490 Klokkarstua (NO)
(72) Inventor: KARLSEN, Frank, N-3490 Klokkarstua (NO); MORLAND, Einar, N-3490 Klokkarstua (NO); MORLAND, Geir, N-3490 Klokkarstua (NO); FALANG, Bente, N-3474 Åros (NO)
(74) Representative: Boult Wade Tennant
(86) International application number: PCT/EP2014/062962
(87) International publication number: WO 2014/202732

(56) References cited:
- EP-A1- 1 997 914
- WO-A1-2005/083129
- US-A1- 2006 172 285
- D. C. RIJKAART ET AL: "High-Risk Human Papillomavirus (hrHPV) E6/E7 mRNA Testing by PreTect HPV-Proofer for Detection of Cervical High-Grade Intraepithelial Neoplasia and Cancer among hrHPV DNA-Positive Women with Normal Cytology", JOURNAL OF CLINICAL MICROBIOLOGY, vol. 50, no. 7, 2 May 2012 (2012-05-02), pages 2390-2396, XP055137206, ISSN: 0095-1137, DOI: 10.1128/JCM.06587-11
- PAOLO GIORGI ROSSI ET AL: "Prognostic Value of HPV E6/E7 mRNA Assay in Women with Negative Colposcopy or CIN1 Histology Result: A Follow-Up Study", PLOS ONE, vol. 8, no. 2, 27 February 2013 (2013-02-27), page e57600, XP055137217, ISSN: 1932-6203, DOI: 10.1371/journal.pone.0057600

### SEQUENCE LISTING

<110> NORCHIP AS
<120> Cervical screening method
<130> P127388WO00
<160> 12
<170> PatentIn version 3.5
<210> 1
   <211> 40
   <212> DNA
   <213> Artificial Sequence
<220>
   <223> Primer
<400> 1
   gatgcaaggt cgcatatgag ccacaggagc gacccagaaa 40
<210> 2
   <211> 51
   <212> DNA
   <213> Artificial Sequence
<220>
   <223> Primer
<400> 2
   aattctaata cgactcacta tagggagaag gattcccatc tctatatact a 51
<210> 3
   <211> 42
   <212> DNA
   <213> Artificial Sequence
<220>
   <223> Primer
<400> 3
   gatgcaaggt cgcatatgag gaaaacgatg aaatagatgg ag 42
<210> 4
   <211> 51
   <212> DNA
   <213> Artificial Sequence
<220>
   <223> Primer
<400> 4
   aattctaata cgactcacta tagggagaag gggtcgtctg ctgagctttc t 51
<210> 5
   <211> 42
   <212> DNA
   <213> Artificial Sequence
<220>
   <223> Primer
<400> 5
   gatgcaaggt cgcatatgag tatcctgaac caactgacct at 42
<210> 6
   <211> 50
   <212> DNA
   <213> Artificial Sequence
<220>
   <223> Primer
<400> 6
   aattctaata cgactcacta tagggagaag gttgacacat aaacgaactg 50
<210> 7
   <211> 41
   <212> DNA
   <213> Artificial Sequence
<220>
   <223> Primer
<400> 7
   gatgcaaggt cgcatatgag aaccattgaa cccagcagaa a 41
<210> 8
   <211> 53
   <212> DNA
   <213> Artificial Sequence
<220>
   <223> Primer
<400> 8
   aattctaata cgactcacta tagggagaag gtctttcttg ccgtgcctgg tca 53
<210> 9
   <211> 32
   <212> DNA
   <213> Artificial Sequence
<220>
   <223> Probe
<400> 9
   ccagcttatg actttgcttt tcgggaagct gg 32
<210> 10
   <211> 33
   <212> DNA
   <213> Artificial Sequence
<220>
   <223> Probe
<400> 10
   cgcatggaac cacaacgtca cacaatgcat gcg 33
<210> 11
   <211> 35
   <212> DNA
   <213> Artificial Sequence
<220>
   <223> Probe
<400> 11
   ccaagcggac aagcacaacc agccacagcg cttgg 35
<210> 12
   <211> 32
   <212> DNA
   <213> Artificial Sequence
<220>
   <223> Probe
<400> 12
   cgatcggtac cgagggcagt gtaatacgat cg 32

## Description

### Field of invention

The invention is concerned with a quality control method for cervical screening of human female subjects.

### Background

Cervical carcinoma is one of the most common malignant diseases worldwide and is one of the leading causes of morbidity and mortality among women. The current conception of cervical carcinoma is that it is a multistage disease, often developing over a period of 10-25 years. The clinical course of cervical carcinoma shows considerable variation; some patients with less favourable tumour characteristics have a relatively good outcome, while others suffer a fatal outcome of an initially limited disease.

It is widely accepted that early identification of cancerous or pre-cancerous cells in the cervix greatly improves the likelihood that any treatment or other intervention will succeed and leads to an improved prognosis. To this end, cervical screening programs have been introduced in many countries.

The gold standard of cervical pre-cancer diagnosis is morphological evaluation of biopsies from the cervix. However, histological assessments of cervical biopsies have their shortcomings; for example, they require complex diagnostic interpretation and are highly invasive. As such, primary cervical screening using cytological methods have been developed, for example the Pap test.

Cytological evaluations such as the Pap test are less invasive than cervical biopsy and allow an initial assessment of cervical cells to be made. If the Pap smear from the subject is deemed by the cytologist, pathologist or gynaecologist doing the screening to exhibit abnormal or atypical cell changes, the subject is considered to be at risk and referred for clinical histological investigation. Cervical screening in this way means only those subjects deemed to be at risk following cytological analysis are referred for cervical biopsy.

However, cytological cervical screening methods such as the Pap test are flawed. It is thought that approximately 50% of women with high-grade cervical dysplasia (CIN2+) are given a "normal" Pap smear result. This may be because assessment of the Pap smears by the cytologist/pathologist/gynaecologist inevitably has a subjective element. Firstly, cytological changes must be identified and, secondly, a judgement must be made as to whether any changes observed are within the normal ranges expected for healthy cells.

Human papillomavirus (HPV) infection is required, but not sufficient for the development of cervical cancer. The main discovery made by Harald zur Hausen, the Nobel Prize winner in Medicine in 2008, was that the real driver of cervical dysplasia and cancer development is the production of E6 and E7 proteins following expression of E6/E7 mRNA from carcinogenic HPV types. The cause of cervical pre-cancer leading to invasive cervical cancer is the continuous production of E6 and E7 proteins following the presence of abnormal E6/E7 mRNA expression.

A number of studies have explored the potential role of HPV testing in cervical screening (see Cuzick et al. A systematic review of the role of human papillomavirus testing within a cervical screening programme. Health Technol Assess 3:14. 1999). Investigation is ongoing into the use of HPV testing in cervical screening as a method of triage or primary identification.

Rijkaart et al, J Clin Microbiol. 2012 Jul;50(7):2390-6 describes E6/E7 mRNA testing for detection of cervical high-grade intraepithelial neoplasia and cancer among high risk HPV DNA-positive women with normal cytology.

Giorgi Rossi et al. PLoS One. 2013;8(2):e57600 describes the prognostic value of HPV E6/E7 mRNA assay in women with negative colposcopy or CIN1 histology result.

### Summary of invention

Current cervical screening methods using cytology to identify subjects with high-grade cervical dysplasia (CIN2+) have a low sensitivity rate: for example, approximately 50% of women with high-grade dysplasia are given a normal Pap test result.

It has been identified that, if "normal" Pap smears from women who went on to develop cervical cancer are reassessed, abnormal or atypical cell changes can be identified that were overlooked at the time of screening.

The assessment of cervical cells from Pap smears, liquid-based samples or colposcopy images by the cytologist, pathologist or gynaecologist requires any cytological or morphological changes to be noticed and identified by the assessor and a subjective judgement made as to the severity of said changes.

As such, at least part of the low sensitivity of such cervical screening methods is due to abnormal or atypical changes in cells of the cervix being over-looked by the cytologist, pathologist or gynaecologist performing the cervical cytological evaluation. Such cell changes may be over-looked because, for example, they were not noticed on first assessment, or because they were subjectively judged as within expected ranges and could therefore be considered as normal. Quality control methods for cervical screening have therefore been devised.

Obligatory repeat cytological cervical evaluation of all subjects would lead to an increase in cost, complexity and time taken for assessment, with no guarantee that identification of abnormal or atypical cell changes would be improved. No quality control effect would be seen as the error inherent in the subjective element of the assessment would be maintained.

Instead, testing a cervical cell sample from a subject for expression of E6/E7 mRNA transcripts from HPV allows identification of subjects whose cervical cytological evaluation can be most effectively repeated. This more focussed and directed approach in selecting subjects for repeat cervical cytological evaluation increases the likelihood that the repeat cervical cytological evaluation will identify previously overlooked abnormal or atypical cell changes and thus improve the quality of the cervical screening.

Therefore, in a first aspect the invention provides a quality control method for cervical screening, the method comprising:
i) testing a cervical cell sample from a human female subject, who has undergone cervical cytological evaluation and was assessed as normal, for expression of mRNA transcripts from the E6/E7 gene of at least one cancer-associated HPV type; and
ii) selecting the subject for repeat cervical cytological evaluation if expression of said mRNA transcripts from the E6/E7 gene of at least one cancer-associated HPV type is detected in said cervical cell sample; and
iii) conducting said repeat cervical cytological evaluation of the subject, wherein the repeat cervical cytological evaluation is to be conducted on the same cervical cell sample or image of cervical cells as the initial cervical cytological evaluation.

This method involves testing a cervical cell sample, taken from a subject who has previously undergone cervical cytological evaluation, for the expression of E6/E7 mRNA from at least one cancer-associated HPV type. If the cervical cell sample has expression of E6/E7 mRNA, the subject is identified as one who should have their cervical cytological evaluation repeated.

By only selecting for repeat cervical cytological evaluation those subjects with cervical cell samples in which expression of E6/E7 mRNA has been detected, this method will increase the likelihood of the re-evaluation identifying previously overlooked abnormal or atypical cell changes, thus improving the quality of the cervical screening.

Many cervical screening programs require any subject with an abnormal cervical cytological evaluation result, such as an abnormal Pap smear, to be referred automatically for further clinical investigation: for example, histological examination. However, subjects with "normal" results may not be put through any further cervical screening or investigation for a number of years. If these subjects in fact have abnormal or atypical cell changes in the cervix that were overlooked, the delay in identification may severely impact the effectiveness of treatment and prognosis. Methods according to aspects of the invention will reduce the likelihood of subjects with abnormal or atypical cell changes in the cervix being miscategorised in this way, i.e. cervical screening using methods according to the invention will have increased sensitivity for detecting abnormal or atypical cell changes in the cervix compared to cervical screening employing a single cervical cytological evaluation.

Reassessment by the cytologist, pathologist or gynaecologist of the same cervical cell sample or image with knowledge that expression of E6/E7 mRNA has been detected in a cervical cell sample from the subject will increase the likelihood that previously-overlooked abnormal or atypical cells will be indentified but does not require further images or samples, thus avoiding further discomfort on the part of the subject or further expense.

The invention will improve the quality of cervical screening beyond that achieved with a single cervical cytological evaluation alone. By focussing cervical cytological re-evaluation on subjects who have had expression of E6/E7 mRNA from at least one cancer-associated HPV type detected in their cervical cell sample, cervical screening employing a quality control method according to the invention will have a greater sensitivity for identifying those subjects having abnormal or atypical cell changes in the cervix.

Cervical screening programs using the invention increase the likelihood that any abnormal or atypical cell changes in the cervix of a subject are identified and increase the proportion of subjects referred for cervical biopsy and histological investigation who are subsequently found to have cervical dysplasia or neoplasia.

### Definitions

"Cervical screening" is a method of identifying female human subjects with cancerous or pre-cancerous cell changes in the cervix and who should therefore be referred for clinical follow-up. Cervical screening may be opportunistic or organised in local, regional or national screening programs.

"Cervical cytological evaluation" is the assessment by e.g. a pathologist/cytologist/gynaecologist of cells of the cervix of a human female subject. The cells of the cervix assessed may be a cervical cell sample, such as that collected for a Pap test. Alternatively, an image or photograph of the cells of the cervix may be assessed, such as that taken during colposcopy or other visual inspection of the cervix (e.g. visual inspection with acetic acid (VIA)). The assessment may be by light microscopy, thin-layer cytology or other cytological or colposcopic techniques known in the art.

Cervical cell samples suitable for cervical cytological evaluation include (but not exclusively) cervical swabs, cervical scrapings, samples removed with the use of brushes, lavage, scrapings, swabs and tampons etc., liquid-based cytology samples, non-liquid based cytology samples, also paraffin embedded tissues, and formalin, methanol or ethanol fixed cells.

A cervical cell sample for cervical cytological evaluation may be collected by a gynaecologist, GP, nurse, other healthcare personnel or by the subject herself using a self-collection device.

The result of cervical cytological evaluation may be normal. A normal result means the cervical cells are assessed as within normal limits and probably not containing any disease. Alternatively, the result may be abnormal. An abnormal result means the cervical cells are assessed as having abnormal or atypical cell changes, for example visual cell changes, cytological changes, morphological changes or other atypical changes related to cervical disease.

Abnormal cervical cytological results include (but not exclusively) those graded as having atypical squamous cells of uncertain significance (ASC-US), low-grade squamous intraepithelial lesion (LSIL), high-grade squamous intraepithelial lesion (HSIL), squamous cell carcinoma and/or adenocarcinoma *in situ* (AIS).

Subjects with abnormal results are referred for clinical follow-up and investigation according to local guidelines for cervical screening.

A method of "quality control" for cervical screening is therefore a method by which the identification of subjects with abnormal cervical cytological evaluation results may be "improved".

Improvement in identification of subjects with abnormal cervical cytological evaluation results as a consequence of the quality control method can be the identification in a cervical cell sample or image of previously over-looked atypical or abnormal cell changes.

Alternatively, improvement in identification of subjects with abnormal cervical cytological evaluation results as a consequence of the quality control method can be the upgrading of a previous cervical cytological evaluation result, e.g. a cervical cytological evaluation result previously graded as LSIL is upgraded to HSIL.

A cervical cell sample to be tested for expression of E6/E7 mRNA transcripts must comprise at least one cervical cell or tissue of a type which is susceptible to infection with human papillomavirus, e.g. cervical epithelial cells. The cervical cell sample to be tested for expression of E6/E7 may be selected to include cell types/tissues which allow testing for expression of E6/E7 mRNA expression in the cervical mucosa and/or the upper layers of cervical epithelium. Cervical cell samples suitable for testing for expression of E6/E7 mRNA include (but not exclusively) cervical swabs, cervical biopsies, cervical scrapings, samples removed with the use of brushes, lavage, scrapings, swabs and tampons etc., skin biopsies/warts, liquid-based cytology samples, also paraffin embedded tissues, and formalin, methanol or ethanol fixed cells.

"Cancer-associated" HPV type means a type of human papillomavirus known to be associated with cancer of the cervix. Cancer-associated HPV types include those "carcinogenic" HPV types classified as carcinogenic (in relation to cancer of the cervix) by the International Agency for Research on Cancer (IARC) monograph.

In particular, cancer-associated HPV types include HPV types 5, 6, 8, 11, 16, 18, 26, 31, 33, 35, 39, 45, 51, 52, 53, 56, 58, 59, 66, 68, 70, 73 and 82.

The terms "mRNA transcripts from the E6/E7 gene" and "E6/E7 mRNA transcripts" are used interchangeably and refer to mRNA transcripts which are transcribed from and contain all or part of the E6 open reading frame, including naturally-occurring splice variants, and mRNA transcripts which are transcribed from and contain all or part of the E7 open reading frame, preferably wherein said mRNA transcripts are naturally occurring.

The term "full-length E6/E7 mRNA transcripts" means any E6/E7 mRNA transcript which encodes a full-length E6 protein. This definition excludes any of the naturally occurring splice variants, but encompasses bicistronic transcripts that encode functional full-length E6 and E7 proteins.

Expression of E6/E7 mRNA transcripts is "detected" in a cervical cell sample if E6/E7 mRNA transcripts are detected at a level above background for the assay or methodology used. Suitable methodologies for testing for expression of E6/E7 mRNA transcripts are well-known to the person skilled in the art, and techniques for determining the background for such assays is well-known and routine for the skilled person. Such methods include mRNA amplification-based techniques, for example RT-PCR, NASBA, TMA, rolling circle amplification etc. or techniques based on hybridisation.

A test having "independent read-outs" has a plurality of read-outs, each indicating the expression of E6/E7 mRNA from one or more HPV types of a specified group. In one non-limiting embodiment, a test with independent read-outs for (i) HPV 16, and (ii) HPV types 18, 33 and 45 can indicate the expression of HPV 16 E6/E7 mRNA specifically, and/or the expression of E6/E7 mRNA from one or more of HPV types 18, 33 and 45. In this embodiment, when read-out (ii) indicates expression of E6/E7 mRNA from one or more of HPV types 18, 33 and 45, no further indication is provided as to from which one or more of the HPV types in the group the E6/E7 mRNA transcripts are derived.

In another embodiment, a test with independent read-outs for (i) HPV 16, and (ii) HPV types 18 and 45 can indicate the expression of HPV 16 E6/E7 mRNA specifically, and/or the expression of E6/E7 mRNA from one or more of HPV types 18 and 45. In this embodiment, when read-out (ii) indicates expression of E6/E7 mRNA from one or more of HPV types 18 and 45, no further indication is provided as to from which one or more of the HPV types in the group the E6/E7 mRNA transcripts are derived.

The following embodiments relate to each and all of the above aspects and embodiments, alone or in combination with any one or more other embodiments, unless otherwise specified.

In one embodiment, cervical cytological evaluation of a subject may be performed on cells of the cervix collected in a cervical cell sample. Cervical cell samples suitable for cervical cytological evaluation include, for example, cervical swabs, cervical scrapings, samples removed with the use of brushes, lavage, scrapings, swabs and tampons etc., liquid-based cytology samples, non-liquid based cytology samples, also paraffin embedded tissues, and formalin, methanol or ethanol fixed cells. In a further embodiment, the cervical cell sample is that collected for a Pap test. In an alternative embodiment, the cervical cell sample is a liquid-based cytology sample.

In one embodiment, the assessment of the cells of the cervix during cervical cytological evaluation is by light microscopy. In a further embodiment, the assessment is by thin-layer cytology.

In an alternative embodiment, cervical cytological evaluation may be performed on an image of the cells of the cervix. In a further embodiment, the image of the cells of the cervix is a photograph. In an alternative embodiment, the image of the cells is a real-time image. In a further embodiment, the image is a colposcopy image. In an alternative embodiment, the image is that from another method of visual inspection of the cervix, for example visual inspection with acetic acid (VIA).

In one embodiment, the method used for the initial cervical cytological evaluation of methods according to the invention is identical to the method used for any subsequent cytological cervical evaluation undertaken as a result of expression of E6/E7 mRNA transcripts from one or more cancer associated HPV types having been detected in a cervical cell sample from the subject.

It is particularly advantageous if the initial cervical cytological evaluation is carried out using liquid-based cytology as the liquid-based cytology sample taken for cervical cytological evaluation can also be tested for E6/E7 mRNA expression. In an alternative embodiment, the initial cervical cytological evaluation is be carried out using non-liquid-based cytology. In this embodiment, it is particularly advantageous if the cervical cell sample to be tested for expression of E6/E7 mRNA is taken separately but concurrently with the cytology sample, e.g. over the course of a single appointment with a gynaecologist.

The cervical cell sample must comprise at least one cervical cell or tissue of a type which is susceptible to infection with human papillomavirus, e.g. cervical epithelial cells. The cervical cell sample to be tested for expression of E6/E7 mRNA may be selected to include cell types/tissues which allow testing for expression of E6/E7 mRNA in the cervical mucosa and/or the upper layers of cervical epithelium. Suitable cervical samples include (but not exclusively) cervical swabs, cervical biopsies, cervical scrapings, samples removed with the use of brushes, lavage, scrapings, swabs and tampons etc., skin biopsies/warts, liquid-based cytology samples, also paraffin embedded tissues, and formalin, methanol or ethanol fixed cells.

The assay for E6/E7 mRNA expression may be carried out on a preparation of nucleic acid isolated from the cervical sample. The preparation of nucleic acid must include mRNA, however it need not be a preparation of purified poly A+ mRNA and preparations of total RNA or crude preparations of total nucleic acid containing both RNA and genomic DNA, or even crude cell lysates may also be used.

E6/E7 mRNA expression may be detected using any suitable methodology. In all embodiments of the methods of the invention, the step of testing a cervical cell sample for expression of E6/E7 mRNA transcripts from at least one cancer-associated HPV type may be carried out using assay methodology already known in the art, including mRNA amplification-based techniques such as for example RT-PCR, NASBA, TMA, rolling circle amplification etc. or techniques based on hybridisation. Suitable techniques are also described in WO 03/057914 and WO 2005/083129.

In the methods of the invention, a decision is made based on expression of E6/E7 mRNA transcripts of at least one cancer-associated HPV type being detected in a cervical cell sample. In this context, expression of E6/E7 mRNA transcripts is "detected" in a cervical cell sample if E6/E7 mRNA transcripts are detected at a level above background for the assay or methodology used. There is no absolute requirement for accurate quantitative determination of the level of E6/E7 mRNA expression, although in certain embodiments, the methods of the invention may comprise a quantitative determination of levels of mRNA expression.

In certain embodiments, subjects may be tested for expression of E6/E7 mRNA transcripts from any one or more of HPV 16, HPV 18, HPV 33 and HPV 45, and optionally one or more additional cancer-associated HPV types, for example one or more selected from the group consisting of HPV types 5, 6, 8, 11, 16, 18, 26, 31, 33, 35, 39, 45, 51, 52, 53, 56, 58, 59, 66, 68, 70, 73 and 82. In such embodiments, subjects are selected for repeat cervical cytological evaluation if expression of E6/E7 mRNA transcripts of any one of the HPV types tested is detected in their cervical cell sample, for example any one of HPV 16, HPV 18, HPV 33 or HPV 45, optionally one or more additional cancer-associated HPV types, for example one or more selected from the group consisting of HPV types 5, 6, 8, 11, 16, 18, 26, 31, 33, 35, 39, 45, 51, 52, 53, 56, 58, 59, 66, 68, 70, 73 and 82.

In certain embodiments, cervical cell samples may be tested for expression of E6/E7 mRNA transcripts from HPV types 16, 18, 33 and 45 only. In such embodiments, subjects are selected for repeat cervical cytological evaluation if expression of E6/E7 mRNA from any one of HPV types 16, 18, 33 and 45 is detected in their cervical cell sample. In further embodiments, subjects may be tested for expression of E6/E7 mRNA transcripts from HPV types 16, 18 and 45 only. In such embodiments, subjects are selected for repeat cervical cytological evaluation if expression of E6/E7 mRNA from any one of HPV types 16, 18, and 45 is detected in their cervical cell sample.

When subjects with invasive cervical cancer were tested with the PreTect HPV-Proofer™ assay (Norchip™), HPV types 16, 18, 33 and 45 together accounted for approximately 91% of cases of invasive cervical cancer. Specifically testing for E6/E7 mRNA from only these types therefore takes advantage of the strong association of these types with cervical cancer and the broad coverage provided, whilst keeping down the complexity and cost of the methods. Similarly, HPV types 16, 18 and 45 together accounted for 88% of invasive cervical cancer cases tested with PreTect HPV-Proofer™. Thus, specifically testing for E6/E7 mRNA from only HPV types 16, 18 and 45 maintains the strong association with invasive cervical cancer cases and broad coverage but further reduces the cost and complexity of the test. In certain embodiments, based on these specific combinations of HPV types, cervical screening employing a quality control method according to the invention will have over 90% sensitivity, for example approximately 90%, 95%, or 100% sensitivity in identification of subjects with abnormal cervical cytological evaluation results, whilst maintaining the high specificity of cytology.

Certain HPV types exhibit a marked geographical or population distribution. Therefore, it may be appropriate to also test for E6/E7 mRNA expression from an HPV type known to be prevalent in invasive cancer in the population/geographical area under test, for example in addition to screening for HPV types 16, 18, and 45, with or without HPV 33.

In certain embodiments in which cervical cell samples are tested for expression of E6/E7 mRNA transcripts from HPV types 16, 18, 33 and 45 only, the assay may have a plurality of independent read-outs. "Read-out" in this case refers to the indication provided by any particular assay used to test for expression of E6/E7 mRNA that E6/E7 mRNA from one or more HPV types has been detected.

In certain embodiments in which the cervical cell sample is tested for expression of E6/E7 mRNA transcripts from HPV types 16, 18, 33 and 45 only, the testing has independent read-outs for (i) HPV 16, and (ii) HPV 18, 33 and 45. In such embodiments, the read-out indicating the expression of E6/E7 mRNA from HPV 16 is independent from the read-out indicating the expression of E6/E7 mRNA from one or more of HPV types 18, 33 and 45. In certain such embodiments, the read-out indicating the expression of E6/E7 mRNA from one or more of HPV types 18, 33 and 45 may provide indication that E6/E7 mRNA has been detected from at least one of those types, but may provide no further indication as to which type or types in particular the E6/E7 mRNA is from.

Similarly, in certain embodiments in which cervical cell samples are tested for expression of E6/E7 mRNA transcripts from HPV types 16, 18 and 45 only, the testing has independent read-outs for (i) HPV 16, and (ii) HPV 18 and 45. In certain such embodiments, the read-out indicating the expression of E6/E7 mRNA from HPV 16 is independent from the read-out indicating the expression of E6/E7 mRNA from one or more of HPV types 18 and 45. In certain such embodiments, the read-out indicating the expression of E6/E7 mRNA from one or more of HPV types 18 and 45 may provide indication that E6/E7 mRNA has been detected from at least one of those types, but may provide no further indication as to which type or types in particular the E6/E7 mRNA is from.

Suitable independent read-outs will depend on the assay and are within the knowledge of the skilled person. For example, probes directed to E6/E7 mRNA from the specific HPV type or types indicated by the independent read-out may be used. If expression of E6/E7 mRNA from more than one HPV type is indicated by a single independent read-out, consensus and/or degenerate primers may be used, and/or degenerate probes, optionally carrying the same fluorophore.

Unless otherwise stated, the terms "E6/E7 mRNA", "E6/E7 transcripts" as used herein encompass all mRNA transcripts which are transcribed from and contain all or part of the E6 open reading frame, including naturally occurring splice variants, and transcripts which are transcribed from and contain all or part of the E7 open reading frame, preferably wherein said mRNA transcripts are naturally occurring.

In certain embodiments of the invention the cervical cell sample is tested for expression of full length E6/E7 mRNA transcripts from at least one cancer-associated HPV type, which transcripts encode a full length E6 protein. In these embodiments specific detection of the full length E6/E7 mRNA is taken as a positive result.

In a further embodiment, the cervical cell sample is tested for expression of full length E6/E7 mRNA transcripts from any one or more of HPV 16, 18, 33, 45 and optionally one or more additional cancer-associated HPV types. In a further embodiment, the cervical cell sample is tested for expression of full length E6/E7 mRNA transcripts from HPV types 16, 18, 33 and 45 only. In a further embodiment, the cervical cell sample is tested for expression of full length E6/E7 mRNA transcripts from HPV types 16, 18, and 45 only.

The term "full length E6/E7 mRNA transcripts" excludes any of the naturally occurring splice variants, but encompasses bicistronic transcripts that encode functional full length E6 and E7 proteins. Four E6/E7 mRNA species have so far been described in cells infected with HPV 16, namely an unspliced E6 transcript and three spliced transcripts denoted E6*I, E6*II and E6*III (Smotkin D, et al., J Virol. 1989 Mar 63(3):1441 7; Smotkin D, Wettstein FO. Proc Natl Acad Sci USA. 1986 Jul 83(13):4680 4; Doorbar J. et al., Virology. 1990 Sep 178(1):254 62; Cornelissen MT, et al. J Gen Virol. 1990 May 71 (Pt 5):1243 6; Johnson MA, et al. J Gen Virol. 1990 Jul 71 (Pt 7):1473 9; Schneider Maunoury S, et al. J Virol. 1987 Oct 61(10):3295 8; Sherman L, et al. Int J Cancer. 1992 Feb 50(3):356 64). All four transcripts are transcribed from a single promoter (p97) located just upstream of the second ATG of the E6 ORF. In the case of HPV 16, the term "full length E6/E7 transcripts" refers to transcripts which contain all or substantially all of the region from nucleotide (nt) 97 to nt 880 in the E6 ORF, inclusive of nt 97 and 880. Nucleotide positions are numbered according to standard HPV nomenclature (see Human Papillomavirus Compendium OnLine, available via the internet or in paper form from HPV Database, Mail Stop K710, Los Alamos National Laboratory, Los Alamos, NM 87545, USA).

In relation to HPV types other than HPV 16, "full length" E6/E7 transcripts may be taken to include transcripts which contain sequences homologous to the above-stated region of the HPV 16 E6/E7 transcript and to exclude E6 splice variants. Various sequence alignments of HPV types are publicly available via the Human Papillomavirus Compendium OnLine.

Specific detection of full length E6/E7 mRNA transcripts (that is detection of full length E6/E7 transcripts in the absence of any spliced E6 transcripts) may be accomplished, for example, using primers or probes which are specific for the region which is present only in full length E6/E7 transcripts, not in splice variants.

The E6*I transcript exhibits loss of a coding sequence between nucleotides 226 and 409 (in HPV type 16) and the E*6II transcript exhibits loss of the coding sequence between nucleotides 226 and 526 (in HPV type 16). It is therefore preferred to use at least one primer or probe from the region located between nucleotides 226 and 409 of HPV type 16 or the homologous region from any other cancer-associated HPV type. Specificity for full length transcripts can be achieved by the use of a primer-pair in which one primer is specific for a sequence located within this region and the other primer is specific for a sequence located outside of this region or wherein both primers are specific for sequences within this region, optionally in conjunction with a probe specific for a sequence located within this region. In other embodiments it may be possible to use a primer-pair in which both primers are specific for sequences outside this region in combination with a probe specific for a sequence within the region in order to confer specificity for mRNA encoding full length E6.

Identification of suitable primers and probes for detecting expression of E6/E7 mRNA transcripts in a cervical cell sample using an amplification technique is within the knowledge of the skilled person. Primer and probes for testing for expression of E6/E7 mRNA transcripts, including full length E6/E7 mRNA transcripts, using NASBA are provided in, for example, WO 2003/057914.

In certain embodiments, the assay used to test for expression of E6/E7 mRNA transcripts has a high analytical sensitivity. In such embodiments, analytical sensitivity is determined as the minimum number of cells infected with the relevant HPV type(s) required in each assay reaction for the assay to detect HPV infection in the reaction sample with 100% sensitivity. Methods for determining analytical sensitivity are known to the skilled person and include, for example, titration assays employing serial dilution of HPV-infected cell lines (e.g. CaSki and SiHa). Suitable methods for determining analytical sensitivity are described in Cuschieri et al. (J Virol Methods, doi: 10.1016/j.jviromet.2013.05.013).

In certain embodiments, the quality control method of the invention can be applied to cervical screening of human female subjects having no previous history of cervical abnormalities. Such subjects could include individuals who have never been enrolled in a cervical screening program and also individuals who have previously been screened using cytological methods, e.g. Pap test or liquid-based cytology, or colposcopy but have never shown any cytological abnormality.

In some instances, the result of the repeat cervical cytological evaluation will be normal, despite mRNA transcripts from the E6/E7 gene being detected in the cervical cell sample. In such instances, it is preferable that a further, new cervical cytological evaluation of the subject be performed within 3-6 months of the most-recent cervical cytological evaluation that gave the "normal" result.

Therefore, in certain embodiments, if the result of the repeat cervical cytological evaluation is assessed as normal, the subject is subjected to further cervical cytological evaluation between 3 months to 6 months from the most-recent cervical cytological evaluation.

In certain embodiments, the human female subject may be 39 years of age or younger. In certain embodiments, the human female subject may be 29 years of age or younger. In an alternative embodiment, the human female subject is 40 years of age or older.

Aspects according to the invention may be particularly advantageous in subjects 39 years or younger, optionally 29 years or younger, as abnormal or atypical cell changes in the cervix are harder to identify. The mean age of patients presenting with invasive cervical cancer associated with one of HPV types 16, 18 or 45 is 47 years, significantly younger than the mean age of patients presenting with invasive cervical cancer associated with other HPV types. A quality control method according to the invention may therefore increase the likelihood of identifying female subjects in these age groups with abnormal or atypical cell changes in the cervix, compared to a single cervical cytological evaluation alone. Such an improvement in the quality of the cervical screening of women 39 years of age or younger, optionally 29 years of age or younger may be greater than the improvement in quality when the methods of the invention are applied to the cervical screening of female subjects 40 years of age or older. Without wishing to be bound by theory, this may be due to abnormal or atypical cell changes in the cervix being more apparent in female subjects 40 years of age or older and therefore less likely to be overlooked in a single cervical cytological evaluation. Quality control methods according to the invention may therefore improve the sensitivity of cervical screening in identifying women 39 years of age or younger, also those 29 years of age or younger, with abnormal or atypical cell changes in the cervix, as well as increase the likelihood of these changes being identified earlier in disease progression.

In certain embodiments, the methods of the invention improve the identification through cervical screening of human female subjects with adenocarcinoma. Adenocarcinoma is often overlooked in a single cervical cytological evaluation as it manifests higher up the endocervical canal and, as a result, abnormal or atypical cell changes may be less apparent in a cervical cell sample and thus more likely to be overlooked. Quality control methods according to the invention will therefore be particularly advantageous in improving identification of subjects with adenocarcinoma or associated pre-cancerous cell changes in the cervix.

Cervical cytological evaluation samples or images graded as having ASC-US or LSIL may be considered as "borderline" rather than necessarily "abnormal" results. Borderline results may be classified as abnormal. Alternatively, a borderline result may be classified as normal. The classification of borderline results as either normal or abnormal may be based on a subjective judgement made by the cytologist, pathologist or gynaecologist in relation to the specific cervical cytological sample or image.

Therefore, in certain embodiments of the invention, improvement in the quality of cervical screening in identification of abnormal or atypical cell changes in the cervix can be due to the cytologist, pathologist or gynaecologist classifying as abnormal a borderline sample previously classified as normal.

As described, a quality control method for cervical screening according to the invention leads to a greater number of subjects identified as having abnormal cervical cytological evaluation results compared to a cervical screening using a single cervical cytological evaluation. This increase in subjects identified as having abnormal results may lead to an increase in subjects referred for further clinical follow-up, for example by histological investigation, and subsequently identified as having histologically confirmed - for example, by cervical biopsy - cervical dysplasia or neoplasia.

Those subjects identified by cervical screening using methods according to the invention but that would have been overlooked by a single cervical cytological evaluation all have had expression of E6/E7 mRNA detected in cells from the cervix. Expression of E6/E7 mRNA transcripts is a strong indicator of the presence of cancerous or pre-cancerous cells in the cervix. Therefore, it is likely that a greater proportion of those subjects identified by methods according to the invention will be shown by histological investigation to have cervical dysplasia or neoplasia, compared to the proportion of those subjects referred for clinical follow-up following cervical screening without the quality control method.

The result of cervical histological investigation is often categorised according to local criteria and guidelines. In one example, a subject is considered as having cervical dysplasia or neoplasia if cervical histological investigation shows cervical intraepithelial neoplasia (CIN). Cervical intraepithelial neoplasia may be categorised as CIN1, CIN2 or CIN3 according to the following criteria:
CIN1: Abnormal lower third of the epithelial layer
CIN2: Abnormal two thirds of the epithelial layer
CIN3: Cell changes in the epithelial layer thickness

In a further example the subject may be considered as having cervical dysplasia or neoplasia if histological analysis shows they have CIN2 or worse. CIN2 or worse may also be termed high-grade cervical dysplasia. In a further example, the subject may be considered as having cervical dysplasia or neoplasia if histological analysis shows they have CIN3 or worse.

Cervical histological investigation may show the subject has invasive cervical dysplasia, in which case the subject is considered to have cancer.

Therefore, quality control methods according to the invention may result in an increase in the number of subjects histologically identified as having cervical dysplasia or neoplasia, when compared to cervical screening without a quality control method according to the invention. Further, methods according to the invention may also result in a higher proportion of the subjects referred to clinic following cervical screening being subsequently shown to have cervical dysplasia or neoplasia, optionally high-grade cervical dysplasia or neoplasia, when compared to cervical screening without a quality control method according to the invention.

Also described is a method of cervical screening, the method comprising the steps of testing a cervical cell sample from a human female subject for expression of mRNA transcripts from the E6/E7 gene from HPV types 16, 18, 33 and 45 only, or HPV types 16, 18, and 45 only, and, if expression of E6/E7 mRNA from any one or more of the stated HPV types is detected in the cervical cell sample, selecting the subject for clinical and/or histological investigation. The cervical cell sample may be tested for expression of full-length E6/E7 mRNA transcripts encoding the full length protein from the stated HPV types, and, if expression of the full length E6/E7 mRNA transcript from any one or more of the stated HPV types is detected in the cervical cell sample, selecting the subject for clinical and/or histological investigation.

Also described is a method of assessing risk of cervical cancer in a subject, the method comprising testing a cervical cell sample from a human female subject for expression of mRNA transcripts from the E6/E7 gene from HPV types 16, 18, 33 and 45 only, or HPV types 16, 18, and 45 only, wherein, if expression of E6/E7 mRNA from any one or more of the stated HPV types is detected in the cervical cell sample, the subject is identified as being at high risk for cervical cancer. The cervical cell sample may be tested for expression of full-length E6/E7 mRNA transcripts encoding the full-length protein from the stated HPV types, and is identified as being at high risk for cervical cancer if expression of full-length E6/E7 mRNA transcripts from any one or more of the stated HPV types is detected.

### Examples

### Quality control methods

Liquid-based cervical cell samples are taken from female human subjects undergoing cervical screening and cells are extracted with the ThinPrep® 2000 (Cytyc Corporation, Marlborough, MA, USA) for cervical cytological evaluation. The test for expression of E6/E7 mRNA expression uses a liquid-based sample from the same material as cervical cytological evaluation.

### Cervical Cytological Evaluation

A cytologist reviews the cervical cell sample and identifies any abnormal or atypical cell changes according to recognised criteria, for example national or local guidelines. If the cervical cytological evaluation result is that the sample is identified as containing abnormal or atypical cell changes, the subject is referred for clinical histological follow-up. If the result is that there are no abnormal or atypical changes, the cervical cell sample is tested for expression of E6/E7 mRNA.

### Detection of expression of E6/E7 mRNA

Cervical cell samples from subjects with normal cytological cervical evaluation results are tested for expression of E6/E7 mRNA transcripts. Nucleic acids are isolated using the automated Nuclisens Extractor (Boom et al., 1990). The material is kept on dry ice (-80°C) and put into 1 ml of lysis buffer followed by 20 seconds of homogenisation using disposable pestles. 100 ml of the sample is further diluted 10 fold in lysis buffer and 100 ml is then extracted for RNA. The extracted RNA is eluted with ∼40 ml of elution buffer (Organon Teknika) and stored at 70oC.

Real time NASBA is performed using the NucliSens Basic Kit (Organon Teknika, Netherlands), intended for the development of user defined RNA amplification assays. The NASBA amplification is carried out in a volume of 20 µl. The primer sets are directed against full length E6/E7 mRNA for HPV types 16, 18, and 45 and optionally HPV type 33.

Primers for detecting these HPV types are:
HPV 16: HPV16.txt 7905 b.p
HPV16P2:
   p2: GATGCAAGGTCGCATATGAGCCACAGGAGCGACCCAGAAA [SEQ ID No. 1]
HPV16 p1
   P1: AATTCTAATACGACTCACTATAGGGAGAAGGATTCCCATCTCTATATACTA [SEQ ID No. 2]
HPV 18: HPV18.txt 7857 b.p
   HPV18P2:
   p2: GATGCAAGGTCGCATATGAGGAAAACGATGAAATAGATGGAG [SEQ ID No. 3]
HPV18P1:
   p1: AATTCTAATACGACTCACTATAGGGAGAAGGGGTCGTCTGCTGAGCTTTCT [SEQ ID No. 4]
HPV 33: HPV33.txt 7909 b.p
HPV33P2:
   p2: GATGCAAGGTCGCATATGAGTATCCTGAACCAACTGACCTAT [SEQ ID No. 5]
HPV33p1
   p1: AATTCTAATACGACTCACTATAGGGAGAAGGTTGACACATAAACGAACTG [SEQ ID No. 6]
HPV45: HPV45.txt 7858 bp
HPV45P2:
   p2: GATGCAAGGTCGCATATGAGAACCATTGAACCCAGCAGAAA [SEQ ID No. 7]
HPV45p1
   p1: AATTCTAATACGACTCACTATAGGGAGAAGGTCTTTCTTGCCGTGCCTGGTCA [SEQ ID No. 8] E6/E7 mRNA transcripts from HPV types 16, 18, 33 and 45 are detected using the following molecular beacon probes:
HPV 16
   H16e6702mb2 ccagctTATGACTTTGCTTTTCGGGAagctgg [SEQ ID No. 9] 5' modification: FAM; 3' modification: BHQ-1
HPV 18
   H18e6702mb1 cgcatgGAACCACAACGTCACACAATGcatgcg [SEQ ID No. 10] 5' modification: CAL; 3' modification: BHQ-2
HPV 33
   H33e6703mb1 ccaagcGGACAAGCACAACCAGCCACAGCgcttgg [SEQ ID No. 11] 5' modification: CAL; 3' modification: BHQ-2
HPV 45
   H45e6701 mb1 cgatcgGTACCGAGGGCAGTGTAATAcgatcg [SEQ ID No. 12] 5' modification: CAL; 3' modification: BHQ-2

(BHQ - Black Hole Quencher; CAL - CalFluor Red 610; FAM - 6 carboxyfluorescein).

Use of these probes indicates through independent read-outs the expression of E6/E7 mRNA transcripts from (i) HPV 16, and (ii) any one or more of HPV 18, 33, and 45.

Use of the above primers provides high analytical sensitivity for the relevant HPV types in the range of 0.1-10 cells/reaction as measured using HPV-infected cell lines.

### Repeat cervical cytological evaluation

Any liquid-based cervical cell samples in which expression of E6/E7 mRNA from HPV 16 and/or any one or more of HPV 18, 33 and 45 is detected are submitted to repeat cervical cytological evaluation. The cytologist reviews the same cervical cell sample as used in the initial cervical cytological evaluation with the knowledge that the subject is positive for expression of E6/E7 mRNA. The additional knowledge of the cytologist in relation to the HPV status of the subject allows the cytologist to identify any abnormal or atypical cell changes in the cervical cell sample that had been initially over-looked.

Cervical cell samples in which expression of E6/E7 mRNA from HPV 16 and/or any one or more of HPV 18, 33 and 45 is not detected are not re-evaluated.

### Cervical screening result

The final cervical cytological evaluation result, whether positive or negative, is reported and determines the follow-up protocol for each subject. Those subjects whose initial cervical cytological evaluation result was normal and who do not have expression of E6/E7 mRNA from HPV 16 and/or any one or more of HPV 18, 33 and 45 detected in their cervical cell sample are not re-evaluated and are reported as having normal cytology.

### Non-liquid-based cytology

If a non-liquid-based cervical cell sample is taken for cervical cytological evaluation, a separate cervical cell sample for mRNA analysis is taken from the subject at the same time and is stored in a transport medium such as ThinPrep® 2000. Cervical cytological evaluation is then performed on the non-liquid-based cervical cell sample by the cytologist. If the result is normal, mRNA is extracted from the cells in the transport medium as described above and tested for E6/E7 transcripts as above described, with cervical cell samples from subjects whose samples have E6/E7 mRNA expression subjected to repeat cervical cytological evaluation, as above described.

### Improved sensitivity

A total of 1618 women less than 40 years of age (25-39 years of age) have undergone cervical screening incorporating a quality control method according to the invention. Around 20 % of these women were identified has having abnormal smears - that is, abnormal or atypical cell changes were identified in the cervical cell sample. Of the approximately 1300 women originally identified as having normal smears, 26 women (2%) were positive for expression of E6/E7 mRNA transcripts from HPV types 16, 18, and 45.

As a result of these 26 women being positive for expression of E6/E7 mRNA transcripts, the original cervical cell samples were reassessed by the pathologist in the knowledge of the positive E6/E7 mRNA result. On reassessment, every one of the original 26 cervical cell samples was reclassified as ASC-US (borderline (abnormal)) by the pathologist.

Of the 26 women identified by the quality control method of the invention, 13 have undergone a follow-up cervical cytological evaluation 3-6 months after the first cervical screening result. Of these 13 women, 10 had abnormal smears at the follow-up cervical cytological evaluation. Of those 10 women, 4 were identified by histology as having CIN3+ neoplasia.

### Methods of assessing risk of cervical cancer

Liquid-based cervical cell samples are taken from female human subjects undergoing cervical screening and nucleic acids extracted as above described. The samples are then tested for expression of E6/E7 mRNA transcripts from HPV types 16, 18, 33 and 45 as above described. If expression E6/E7 mRNA from any one or more of HPV 16, 18, 33 and 45 is detected in a cervical cell sample, the subject from whom the sample was taken is identified as being at high risk of cervical cancer and may be treated or referred for clinical and/or histological follow-up as appropriate according to local guidelines.

## Claims

1. A method of quality control for cervical screening comprising:
i) testing a cervical cell sample from a human female subject, who has undergone cervical cytological evaluation and was assessed as normal, for expression of mRNA transcripts from the E6/E7 gene of at least one cancer-associated HPV type;
ii) selecting the subject for repeat cervical cytological evaluation if expression of said mRNA transcripts from the E6/E7 gene of at least one cancer-associated HPV type is detected in said cervical cell sample; and
iii) conducting said repeat cervical cytological evaluation of the subject,
wherein the repeat cervical cytological evaluation is to be conducted on the same cervical cell sample or image of cervical cells as the initial cervical cytological evaluation.

2. The method of claim 1 wherein the cervical cell sample is tested for expression of full-length E6/E7 mRNA transcripts of at least one cancer-associated HPV type, which transcripts encode a full-length E6/E7 protein.

3. The method of claim 1 or claim 2 wherein the cervical cell sample is tested for expression of E6/E7 mRNA transcripts from any one or more of HPV 16, HPV 18, HPV 33, HPV 45 and optionally one or more additional cancer-associated HPV types.

4. The method of claim 1 or claim 2 wherein the cervical cell sample is tested for expression of E6/E7 mRNA transcripts from HPV types 16, 18, 33 and 45 only.

5. The method of claims 1 or claim 2 wherein the cervical cell sample is tested for expression of E6/E7 mRNA transcripts from HPV types 16, 18 and 45 only.

6. The method of claim 4, wherein the testing for expression of E6/E7 mRNA from HPV types 16, 18, 33 and 45 has independent read-outs for:
i) HPV 16; and
ii) any one or more of HPV 18, 33, and 45.

7. The method of claim 5, wherein the testing for expression of E6/E7 mRNA from HPV types 16, 18 and 45 has independent read-outs for:
i) HPV 16; and
ii) any one or more of HPV 18 and 45.

8. The method of any preceding claim wherein the human subject is 39 years of age or younger.

9. The method of claim 8 wherein the human subject is 29 years of age or younger.

10. The method of claims 1-7 wherein the human subject is 40 years of age or older.

## Patentansprüche

1. Verfahren zur Qualitätskontrolle für Gebärmutterhals-Screening, umfassend:
i) Testen einer Gebärmutterhals-Zellprobe eines humanen weiblichen Subjekts, das einer zytologischen Gebärmutterhals-Bewertung unterzogen wurde und als normal beurteilt wurde, auf Expression von mRNA-Transkripten des E6/E7-Gens von mindestens einem Krebs-assoziierten HPV-Typ;
ii) Auswählen des Subjekts für eine erneute zytologische Gebärmutterhals-Bewertung, falls Expression der mRNA-Transkripte des E6/E7-Gens von mindestens einem Krebs-assoziierten HPV-Typ in der Gebärmutterhals-Zellprobe festgestellt wird; und
iii) Durchführen der erneuten zytologischen Gebärmutterhals-Bewertung an dem Subjekt, wobei die erneute zytologische Gebärmutterhals-Bewertung an derselben Gebärmutterhals-Zellprobe oder an demselben Bild von Gebärmutterhals-Zellen durchgeführt werden soll, wie die ursprüngliche zytologische Gebärmutterhals-Bewertung.

2. Verfahren nach Anspruch 1, wobei die Gebärmutterhals-Zellprobe auf Expression von Vollängen-E6/E7-mRNA-Transkripten von mindestens einem Krebs-assoziierten HPV-Typ getestet wird, wobei die Transkripte für ein Volllängen-E6/E7-Protein kodieren.

3. Verfahren nach Anspruch 1 oder Anspruch 2, wobei die Gebärmutterhals-Zellprobe auf Expression von E6/E7-mRNA-Transkripten von einem oder mehreren von HPV 16, HPV 18, HPV 33, HPV 45 und gegebenenfalls einem oder mehreren zusätzlichen Krebs-assoziierten HPV-Typen getestet wird.

4. Verfahren nach Anspruch 1 oder Anspruch 2, wobei die Gebärmutterhals-Zellprobe nur auf Expression von E6/E7-mRNA-Transkripten von HPV-Typen 16, 18, 33 und 45 getestet wird.

5. Verfahren nach Anspruch 1 oder 2, wobei die Gebärmutterhals-Zellprobe nur auf Expression von E6/E7-mRNA-Transkripten von HPV-Typen 16, 18 und 45 getestet wird.

6. Verfahren nach Anspruch 4, wobei das Testen auf Expression von E6/E7-mRNA von HPV-Typen 16, 18, 33 und 45 unabhängige Auslesungen aufweist für:
i) HPV 16; und
ii) einen oder mehrere von HPV 18, 33 und 45.

7. Verfahren nach Anspruch 5, wobei das Testen auf Expression von E6/E7-mRNA von HPV-Typen 16, 18 und 45 unabhängige Auslesungen aufweist für:
i) HPV 16; und
ii) einen oder mehrere von HPV 18 und 45.

8. Verfahren nach einem der vorhergehenden Ansprüche, wobei das humane Subjekt 39 Jahre alt oder jünger ist.

9. Verfahren nach Anspruch 8, wobei das humane Subjekt 29 Jahre alt oder jünger ist.

10. Verfahren nach den Ansprüchen 1-7, wobei das humane Subjekt 40 Jahre alt oder älter ist.

## Revendications

1. Procédé de contrôle de qualité pour le dépistage du cancer du col de l'utérus, comprenant les étapes consistant à :
i) tester un échantillon de cellules cervicales d'un sujet humain féminin, qui a subi une évaluation cytologique cervicale et a été évalué comme normal, pour l'expression de transcrits d'ARNm à partir du gène E6/E7 d'au moins un type de HPV associé au cancer ;
ii) sélectionner le sujet pour une évaluation cytologique cervicale répétée si l'expression desdits transcrits d'ARNm à partir du gène E6/E7 d'au moins un type de HPV associé au cancer est détectée dans ledit échantillon de cellules cervicales ; et
iii) effectuer ladite évaluation cytologique cervicale répétée du sujet,
dans lequel l'évaluation cytologique cervicale répétée doit être effectuée sur le même échantillon de cellules cervicales ou la même image de cellules cervicales que l'évaluation cytologique cervicale initiale.

2. Procédé selon la revendication 1, dans lequel l'échantillon de cellules cervicales est testé pour l'expression de transcrits d'ARNm de E6/E7 de pleine longueur d'au moins un type de HPV associé au cancer, lesquels transcrits codent pour une protéine E6/E7 de pleine longueur.

3. Procédé selon la revendication 1 ou 2, dans lequel l'échantillon de cellules cervicales est testé pour l'expression de transcrits d'ARNm de E6/E7 à partir d'un quelconque ou plusieurs parmi HPV 16, HPV 18, HPV 33, HPV 45 et éventuellement un ou plusieurs types supplémentaires de HPV associé(s) au cancer.

4. Procédé selon la revendication 1 ou la revendication 2, dans lequel l'échantillon de cellules cervicales est testé pour l'expression des transcrits d'ARNm de E6/E7 uniquement à partir des types de HPV 16, 18, 33 et 45.

5. Procédé selon la revendication 1 ou 2, dans lequel l'échantillon de cellules cervicales est testé pour l'expression de transcrits d'ARNm de E6/E7 uniquement à partir des types de HPV 16, 18 et 45.

6. Procédé selon la revendication 4, dans lequel le test pour l'expression d'ARNm de E6/E7 à partir des types de HPV 16, 18, 33 et 45 a des lectures indépendantes pour :
i) HPV 16 ; et
ii) un quelconque ou plusieurs des HPV 18, 33 et 45.

7. Procédé selon la revendication 5, dans lequel le test pour l'expression d'ARNm de E6/E7 à partir des types de HPV 16, 18 et 45 a des lectures indépendantes pour :
i) HPV 16 ; et
ii) un quelconque ou plusieurs des HPV 18 et 45.

8. Procédé selon l'une quelconque des revendications précédentes, dans lequel le sujet humain a 39 ans ou moins.

9. Procédé selon la revendication 8, dans lequel le sujet humain a 29 ans ou moins.

10. Procédé selon les revendications 1 à 7, dans lequel le sujet humain a 40 ans ou plus.
